# EUROPÄISCHE PATENTANMELDUNG

(11) **EP 2 757 106 A1**
(43) Veröffentlichungstag der Anmeldung: **23.07.2014**
(21) Anmeldenummer: 13152026.4
(22) Anmeldetag: 21.01.2013
(51) Int. Cl.: C07F 9/6506, C07D 333/08, C07B 37/04, C07D 333/54, C07D 209/08, C07D 307/79, C07C 201/12

(54) **Substituierte Benzimidazolyphosphin-Verbindungen sowie ein Verfahren zur decarboxylierenden Kohlenstoff-Kohlenstoff-Verknüpfung**

(71) Anmelder: Saltigo GmbH, 51369 Leverkusen (DE)
(72) Erfinder: Die Erfindernennung liegt noch nicht vor
(74) Vertreter: Pettrich, Klaus-Günter

(57) **Zusammenfassung**

Beschrieben wird ein Verfahren zur Knüpfung von Kohlenstoff-Kohlenstoff Bindungen durch Umsetzung von Carbonsäuresalzen mit Aryl-, Heteroaryl- oder Vinylalkylsulfonaten wie zum Beispiel Methansulfonaten unter Freisetzung von Kohlendioxid in Gegenwart eines bimetallischen Katalysatorsystems mit Benzimidazolylphosphin-Verbindungen als Liganden. Die Erfindung betrifft weiterhin die Benzimidazolylphosphin-Verbindungen und entsprechenden Metallkomplexe sowie Katalysatoren als solche.

## Beschreibung

Die Erfindung betrifft ein Verfahren zur Knüpfung von Kohlenstoff-Kohlenstoff Bindungen durch Umsetzung von Carbonsäuresalzen mit Aryl-, Heteroaryl- oder Vinylalkylsulfonaten wie zum Beispiel Methansulfonaten, die nachfolgend auch Mesylate genannt werden, unter Freisetzung von Kohlendioxid in Gegenwart eines bimetallischen Katalysatorsystems mit Benzimidazolylphosphin-Verbindungen als Liganden. Die Erfindung betrifft weiterhin die Benzimidazolylphosphin-Verbindungen und entsprechenden Metallkomplexe als solche.

Decarboxylierende Kreuzkupplungen haben sich als eine breit einsetzbare Methodik zur regioselektiven Knüpfung von Kohlenstoff-Kohlenstoff- und Kohlenstoff-Heteroatom-Bindungen bewährt ( W. I. Dzik, P. P. Lange, L. J. Gooßen, Chem. Sci. 2012, 3, 2671-2678). Gegenüber traditionellen Kreuzkupplungen haben sie den prinzipiellen Vorteil, dass anstelle empfindlicher und teurer Organometallverbindungen stabile und breit verfügbare Carbonsäuresalze als Kohlenstoff-Nucleophile eingesetzt werden. Zu den C-C-Verknüpfung werden Carbonsäuresalze mit Organohalogeniden ( L. J. Gooßen, G. Deng, L. M. Levy, Science 2006, 313, 662-664) oder Pseudohalogeniden wie Tosylaten oder Triflaten ( L. J. Gooßen, N. Rodriguez, C. Linder, J. Am. Chem. Soc. 2008, 130, 15248-15249) in Gegenwart von Übergangsmetallkatalysatoren umgesetzt. Als Katalysatoren haben sich insbesondere bimetallische Systeme bewährt, die aus Palladiumsalzen und Silber- oder Kupfersalzen erzeugt werden.

Während der Einsatz von Aryliodiden und -bromiden als Kohlenstoffelektrophile in decarboxylierenden Kupplungen mit relativ einfachen Katalysatorsystemen möglich ist, sind für die weniger reaktiven Arylchloride besonders elektronenreiche Ligandensysteme erforderlich. Der Einsatz von Trifluormethansulfonaten oder 4-Toluolsulfonaten als Kohlenstoffelektrophile ist ebenfalls nicht generell möglich, sondern erfordert den Einsatz spezieller elektronenreicher Phosphinliganden.

Unter den wenigen Katalysatoren, die in der Lage sind, die extrem stabile C-O Bindung von Mesylaten zu aktivieren, sind Ni(PCy)₂Cl₂ ( B. M. Rosen, K. W. Quasdorf, D. A. Wilson, N. Zhang, A.-M. Resmerita, N. K. Garg, V. Percec, Chem. Rev. 2011, 111, 1346-1416) sowie Palladiumkomplexe mit ausgefeilten Liganden der X-Phos, Brett-Phos und CM-Phos-Reihen ( D. S. Surry, S. L. Buchwald, Angew. Chem. Int. Ed. 2008, 47, 6338-6361). Dabei konnte gezeigt werden, dass mit solchen Katalysatoren Mesylate als Substrate in traditionellen Kreuzkupplungen wie z. B. Suzuki-Miyaura, Hiyama und Stille-Reaktionen sowie bei Carbonylierungen und C-Heteroatom Bindungsknüpfungen eingesetzt werden können (C. M. So, F. Y. Kwong, Chem. Soc. Rev. 2011, 40, 4963-4972).

Bisher gibt es jedoch noch kein Beispiel für die Verwendung der notorisch unreaktiven Mesylate in decarboxylierenden Kreuzkupplungen. Im Vergleich zu anderen Kohlenstoff-Elektrophilen besitzen Mesylate einige entscheidende Vorteile, die es überaus erstrebenswert machen, sie als Substrate zu erschließen.

Organische Sulfonate sind präparativ einfach zugänglich etwa durch Veresterung von breit verfügbaren Phenolen oder Enolaten mit den entsprechenden Sulfonsäurechloriden oder -anhydriden. Durch die unterschiedliche Herstellungsweise der Startmaterialien sind sie häufig in komplementären Substitutionsmustern zu den entsprechenden Halogenverbindungen verfügbar.

Der besondere Vorteil der Methansulfonate gegenüber anderen gebräuchlichen Sulfonaten, z. B. 4-Toluolsulfonaten und Trifluormethansulfonaten ist, dass sie eine deutlich niedrigere Molmassen besitzen, was ihren Einsatz atomökonomischer und abfallärmer macht und dass die benötigten Sulfonsäurechloride bzw -anhydride kostengünstiger verfügbar sind.

Die Erschließung von Methansulfonaten als Substrate für decarboxylierende Kreuzkupplungen ist daher von hohem wirtschaftlichem Interesse, und es bestand folglich ein Bedarf an einem Katalysatorsystem für decarboxylierende Kreuzkupplungen, das es erlaubt, die stabile C-O Bindung von Mesylaten zu aktivieren.

Aus mehreren Gründen war die Verwendung von Mesylaten jedoch speziell in decarboxylierenden Kreuzkupplungen besonders erschwert. Als Kohlenstoff-Nucleophile dienen bei solchen Reaktionen Organokupfer- beziehungsweise Organosilberspezies, die durch Extrusion von Kohlendioxid aus den entsprechenden Carboxylatkomplexen in katalytischen Mengen in situ generiert werden. Gerade ausgesprochen elektronenreiche Liganden, wie sie zur Mesylat-Aktivierung eingesetzt werden, vermindern aber die Decarboxylierungsaktivität von Silber- und Kupferkomplexen (L. J. Gooßen, N. Rodriguez, C. Linder, P. P. Lange, A. Fromm, ChemCatChem 2010, 2, 430-442.).

In experimentellen Studien bestätigte sich tatsächlich, dass sich durch Kombination von Kupferkomplexen, die bekanntermaßen die Decarboxylierung von Carbonsäuren vermitteln (L. J. Gooßen, N. Rodriguez, C. Linder, P. P. Lange, A. Fromm, ChemCatChem 2010, 2, 430-442) mit Nickelkatalysatoren, wie sie zur Mesylat-Aktivierung beschrieben sind, kein wirksames Katalysatorsystem für die decarboxylierende Kreuzkupplung von Arylmesylaten ergibt. Auch durch die Kombination von Palladiumkatalysatoren, die bekanntermaßen in die C-O Bindung von Arylmesylaten insertieren können, mit kupferbasierten Decarboxylierungskatalysatoren (Cu₂O/1,10-Phenanthrolin) ergibt sich kein effizientes Katalysatorsystem für die decarboxylierende Kreuzkupplung. Auch die von Kwong et al. erfolgreich in Suzuki-Miyaura-Kupplungen, Cyanierungen, Heck-Mizoroki-Reaktionen und verwandten Reaktionen (C. M. So, F. Y. Kwong, Chem. Soc. Rev. 2011, 40, 4963-4972) eingesetzten Palladiumkomplexe mit Phenylindolphosphinliganden (CM-Phos) sind für decarboxylierende Kreuzkupplungen mit Mesylaten unvorteilhaft. Auch nach intensiver Optimierung der Reaktionsbedingungen wurden mit CM-Phos lediglich unbefriedigende Ausbeuten von wenigen Prozent erhalten.

Strukturell einfache Vertreter von 1,2-substituierter Benzimidazolylphosphin-Palladium-Komplexe wurden bereits von Kwong in Suzuki-Miyaura-Kupplungen von sterisch anspruchslosen Arylboronsäuren mit Arylmesylaten und Arylchloriden eingesetzt, ohne dass sich dabei aber besondere Vorteile dieser Ligandenklasse gegenüber strukturell verwandten Liganden zeigten. Im direkten Vergleich mit CM-Phos waren die Benzimidazolylphosphin-Liganden unterlegen, da sie nicht über die breite Anwendbarkeit des CM-Phos verfügen. (siehe hierfür: a) K. H. Chung, C. M. So, S. M. Wong, C. H. Luk, Z. Zhou, C. P. Lau, F. Y. Kwong, Chem. Commun. 2012, 48, 1967-1969; b) S. M. Wong, C. M. So, K. H. Chung, C. P. Lau, F. Y. Kwong, Eur. J. Org. Chem. 2012, 4172-4177).

Es ist somit deutlich erkennbar, dass auch für den Fachmann unvorhersehbar ist, ob und welche Palladiumkomplexe in Kombination mit welchen Kupfer- oder Silberkomplexen eine decarboxylierende Kreuzkupplung von Organylmesylaten mit Carbonsäuresalzen erlauben.

Aufgabe der vorliegenden Erfindung war es daher, Katalysatoren aufzufinden, welche eine Kohlenstoff-Kohlenstoff-Verknüpfung zwischen Carbonsäuren bzw. Carbonsäuresalzen und Aryl-,Heteroaryl- bzw. Vinylalkylsulfonaten wie insbesondere -mesylaten in effizienter und einfach durchzuführender Weise ermöglichen.

Überraschend wurde nun in Erfüllung der Aufgabenstellung ein Katalysatorsystem gefunden, das die Kreuzkupplung einer breiten Vielfalt von Aryl-, Heteroaryl-und Vinylalkylsulfonaten wie insbesondere - mesylaten mit Carbonsäuren bzw. Carbonsäuresalzen effizient vermittelt.

Die Erfindung betrifft daher ein Verfahren zur Herstellung von Verbindungen der Formel (III)

R¹-R^{X} (III)

In der R¹ für Wasserstoff oder einen über ein sp2-hybridisiertes Kohlenstoffatom gebundenen unsubstituierten oder substituierten Rest aus der Gruppe C₆-C₂₀-Aryl, C₃-C₂₀-Heteroaryl oder C₂- C₂₀- Alkenyl steht,
und
_{R}^{X} für 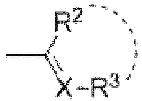 steht, worin X wahlweise für ein Stickstoffatom oder für einen Rest C-R⁴ steht und
R³ für eine organischen Rest aus der Gruppe C₁-C₁₀-Alkyl, C₂-C₂₀- Alkenyl-, unsubstituiertes oder substituiertes C₆-C₂₀-Aryl oder für unsubstituiertes oder substituiertes Heteroaryl aus der Gruppe Pyridin, Pyrimidin, Pyridazin, Pyrazin, Triazin, Tetrazin, Pyrrol, Pyrazol, Isoxazol, Imidazol, Oxazol, Thiazol, Thiophen, Furan, steht und
R² und R⁴ unabhängig voneinander für Wasserstoff stehen oder die gleiche Bedeutung, wie für R³ angegeben, besitzen
oder R² und X-R³ Bestandteil eines unsubstituierten oder substituierten (hetero)cyclischen Ringsystems aus der Gruppe Benzol, Naphtalin, Dihydronaphtalin wie z.B. 1,2-Dihydronaphtalin, Cyclopent-1-en, Phenanthren, Pyridin, Pyrimidin, Pyridazin, Pyrazin, Triazin, Tetrazin, Pyrrol, Pyrazol, Isoxazol, Imidazol, Oxazol, Thiazol, Thiophen, und Furan sind,
durch Umsetzung von Verbindungen der Formel (I)

R¹-COOM (I)

in der M für ein positiv geladenes Ion aus der Gruppe der Alkalimetalle Li, Na, K, Rb, Cs, oder ein Ammoniumkation der Form ⁺NR¹⁶R¹⁷R¹⁸R¹⁹ steht, bei der R,¹⁶R,¹⁷R¹⁸ und R¹⁹ gleich oder verschieden für Wasserstoffatome oder C₁-C₂₀-Alkyl stehen, und
R¹ die für Formel (III) angegebene Bedeutung besitzt,
mit Verbindungen der Formel (II)

R^{X}-O-SO₂-ALK (II)

in der ALK für C₁-C₂₀-Alkyl, vorzugsweise Methyl steht
und
R^{x} die für Formel (III) angegebene Bedeutung besitzt,
in Gegenwart eines Katalysators, der enthält
- zumindest einen Kupferkomplex, enthaltend ein Amin, oder alternativ zumindest einen Silberkomplex, enthaltend ein Amin , und
- zumindest einen Palladiumkomplex, vorzugsweise in den Oxidationsstufen (II) oder (0), enthaltend einen Benzimidazolylphosphin-Liganden, gemäß Formel (IV). 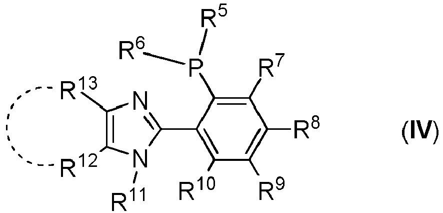
in welcher
R⁵ und R⁶ unabhängig voneinander stehen für einen Rest aus der Gruppe C₁-C₂₀-Alkyl, C₂-C₂₀-Vinyl-, C₆-C₂₀-Aryl-, oder C₃-C₂₀-Heteroaryl, C₁-C₂₀-Alkoxy, C₆-C₂₀-Aryloxy, (C₁-C₂₀-Alkyl)-amino, (C₆-C₂₀-Aryl)amino, Di-(C₆-C₂₀-Aryl)amino oder
R⁵ und R⁶ gemeinsam für C₂-C₁₈-Alkandiyl stehen und
R⁷, R⁸, R⁹, R¹⁰ unabhängig voneinander für einen Rest aus der Gruppe Wasserstoff, Formyl, Hydroxy, Amino, Cyano, Nitro, Fluor, Chlor, Brom, Iod, C₁-C₂₀-Alkyl, C₂-C₂₀-Vinyl-, C₆-C₂₀-Aryl-, oder C₃-C₂₀-Heteroaryl stehen und
R¹¹ für einen Rest aus der Gruppe Wasserstoff, C₁-C₂₀-Alkyl, C₂-C₂₀-Vinyl-, C₆-C₂₀-Aryl-, oder C₄-C₂₀-Heteroaryl und
R¹² und R¹³ unabhängig voneinander für einen Rest aus der Gruppe Wasserstoff, Carboxy, Cyano, Nitro, Fluor, Chlor, Brom, Iod, C₁-C₂₀-Alkyl, C₂-C₂₀-Vinyl-, C₆-C₂₀-Aryl-, oder C₃-C₂₀-Heteroaryl stehen oder R¹² und R¹³ gemeinsam Bestandteil eines nichtaromatischen, aromatischen oder heteroaromatischen Ringsystems sind.

Im Rahmen der Erfindung können alle oben stehenden und im Folgenden aufgeführten, allgemeinen oder in Vorzugsbereichen genannten Definitionen der Reste, Parameter und Erläuterungen untereinander, also auch zwischen den jeweiligen Bereichen und Vorzugsbereichen in beliebiger Weise kombiniert werden.

Die Bezeichnungen C₁-C₂₀-Alkyl und C₁-C₈-Alkyl stehen jeweils unabhängig für einen geradkettigen oder bei mindestens drei Kohlenstoffatomen auch cyclischen oder verzweigten Alkylrest.

C₁-C₈-Alkyl steht beispielsweise für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, cyclo-Hexyl, cycloPentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl , n-Octyl und Cyclohexyl und
C₁-C₂₀-Alkyl weiter darüber hinaus beispielsweise für Adamantyl, n-Nonyl, n-Decyl und n-Dodecyl.

Die Bezeichnungen C₁-C₂₀-Alkoxy und C₁-C₈-Alkoxy stehen jeweils unabhängig für einen geradkettigen oder bei mindestens drei Kohlenstoffatomen auch cyclischen oder verzweigten Alkoxyrest.

C₁-C₈-Alkoxy steht beispielsweise für Methoxy, Ethoxy, n-Propoxy, iso-Propoxy, n-Butoxy, sec.-Butoxy und tert.-Butoxy, n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy, n-Hexoxy und n-Octoxy,

C₁-C₂₀-Alkoxy weiter darüber hinaus beispielsweise für Adamantoxy, die isomeren Menthoxy-Reste, n-Decoxy und n-Dodecoxy.

Die Bezeichnung Alkandiyl steht für divalente Reste der Formel CₙH₂ₙ wie z.B. 1,2-Ethandiyl oder 1,3 Propandiyl.

Die Bezeichnungen C₂-C₂₀-Alkenyl und C₂-C₈-Alkenyl stehen jeweils unabhängig für einen geradkettigen oder bei mindestens 3 Kohlenstoffatomen auch verzweigten oder bei mindestens 5 Kohlenstoffatomen auch cyclischen Alkenylrest.

C₂-C₂₀-Alkenyl steht beispielsweise für Vinyl, 1-Propenyl, iso-Propenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 1-Heptenyl, 1-Octenyl oder 2-Octenyl.

C₆-C₂₀-Aryl bezeichnet jeweils einen carbocyclischen, aromatischen Rest mit 6 bis 20 Gerüstkohlenstoffatomen wie beispielsweise Phenyl, Naphtyl, Phenanthrenyl, Anthracenyl oder Fluorenyl.

C₃-C₂₀-Heteroaryl bezeichnet jeweils einen heteroaromatischen Rest mit 3 bis 20 Gerüstkohlenstoff atomen, wobei im gesamten aromatischen System mindestens ein Gerüstatom ein Heteroatom ausgewählt aus der Gruppe Stickstoff, Schwefel oder Sauerstoff ist. C₃-C₂₀-Heteroaryl steht beispielsweise für Pyridin, Pyrimidin, Pyridazin, Pyrazin, Triazin, Tetrazin, Pyrrol, Pyrazol, Isoxazol, Indol, Imidazol, Oxazol, Thiazol, Thiophen, Furan, Benzofuran.

Weiterhin können die Reste C₆-C₂₀-Aryl und C₃-C₂₀-Heteroaryl mit bis zu fünf gleichen oder verschiedenen Substituenten pro Cyclus substituiert sein, die ausgewählt sind aus der Gruppe Chlor, Fluor, Iod, Brom, Nitro, C₁-C₂₀-Alkyl wie z.B. Methyl, C₁-C₈-Alkoxy wie z.B. Methoxy.

R¹ in der Formel (I) kann für ein über einen sp2-hybridisiertes Kohlenstoffatom gebundenen unsubstituierten oder substituierten Rest aus der Gruppe C₆- C₂₀-Aryl oder unsubstituiertes oder substituiertes Heteroaryl aus der Gruppe Pyridin, Pyrimidin, Pyridazin, Pyrazin, Triazin, Tetrazin, Pyrrol, Pyrazol, Isoxazol, Imidazol, Oxazol, Thiazol, Thiophen, Furan oder C₂- C₁₀- Alkenyl stehen.

Beispiele für R¹ ausgewählt aus der Gruppe C₆- C₁₀-Aryl sind z.B. Phenyl oder Naphtyl und Beispiele für für R¹ ausgewählt aus der Gruppe C₃-C₂₀-Heteroaryl sind z.B. Pyridin, Pyrimidin, Pyridazin, Pyrazin, Triazin, Tetrazin, Pyrrol, Pyrazol, Indol, Isoxazol, Imidazol, Oxazol, Thiazol, Thiophen, Furan, Benzofuran, wobei jeder Rest seinerseits weitere Substituenten tragen kann. Beispiele für substituiertes C₆- C₁₀-Aryl für R¹ sind 2-Nitrophenyl, 5-Methyl-2-Nitrophenyl, 5-Metoxy-2-Nitrophenyl, 4,5-Dimetoxy-2-Nitrophenyl, 3-Methyl-2-Nitrophenyl, 2-Methyl-6-Nitrophenyl, 5-Fluor-2-Nitrophenyl, 6-Methyl-2-Nitrophenyl, oder Pentfluorphenyl. Beispiele für substituiertes C₃-C₂₀-Heteroaryl für R¹ sind 3-Methylhiophen-yl, 3-Methylbenzothiophen-yl, 3-Methylbenzofuranyl oder 1-Methyl-1H-Indyl.

Besonders bevorzugt werden im erfindungsgemäßen Verfahren als Verbindungen der Formel (I) die Kaliumsalze der 2-Nitrobenzoesäure, 4-Methyl-2-nitrobenzoesäure, 4-Methoxy-2-Nitrobenzoesäure, 3-Methyl-2-Nitrobenzoesäure, 4-Fluor-2-Nitrobenzoesäure, 6-Methyl-2- Nitrobenzoesäure, 2-Anissäure, 3-Methyl2-thiophencarbonsäure, 3-Methyl-2-benzothiophen-carbonsäure, 3-Methyl-2-benzofuran-carbonsäure, N-Methyl-2-inolcarbonsäure, 3- Nitrobenzoesäure, 4,5-Dimethyoxybenzoesäure, 2,3,4,5,6-Pentafluor-benzoesäure und Zimtsäure eingesetzt.

Die Verbindungen der Formel (I) können wahlweise auch in situ aus den entsprechenden Carbonsäuren mit geeigneten Basen erzeugt werden.

Im erfindungsgemäßen Verfahren werden als Verbindungen der Formel (II) bevorzugt Mesylate eingesetzt, wobei im Rest R^{X} X wahlweise für ein Stickstoffatom oder für einen organischen Rest C- R⁴ steht.

In einer Variante sind die Reste R² und X-R³ in R^{x} gemeinsam Bestandteil eines (hetero)cyclischen Ringsystems aus der Gruppe Benzol, Naphtalin, Dihydronaphtalin, Phenanthren, Pyridin, Pyrimidin, Pyridazin, Pyrazin, Triazin, Tetrazin, Pyrrol, Pyrazol, Isoxazol, Imidazol, Oxazol, Thiazol, Thiophen, Furan, die ihrerseits wahlweise weitere Substituenten tragen können.

Besonders bevorzugt werden Organomesylate der Formel (II) aus der Gruppe 2-Naphthyl, 1-Naphthyl-, 9-Phenanthryl-, 4-Toluyl, 3-Methoxyphenyl, 3-Anisyl, 3,4-Dehydronaphthyl-, 1,1-Diphenylethenyl, Styryl- , Vinyl-, Alkenyl- eingesetzt.

Im erfindungsgemäßen Verfahren wird ein Molverhältnis von 1 bis 10 Äquivalenten zwischen den Verbindungen der Formel (I) und den Verbindungen der Formel (II) gewählt. Bevorzugt ist das Molverhältnis 1:1 bis 1:2 zwischen den Verbindungen der Formel (I) zu den Verbindungen der Formel (II).

Im erfindungsgemäßen Verfahren findet ein Katalysatorsystem Anwendung, das einen kupfer- oder silberbasierten Decarboxylierungskatalysator und einem Palladiumkomplex mit Benzimidazolylphosphin-Liganden als Kreuzkupplungskatalysator enthält, wobei beide Katalysatoren aus Einzelkomponenten im Reaktionsgemisch in einem zusätzlichen Verfahrensschritt hergestellt werden können.

Im erfindungsgemäßen Verfahren beträgt die Menge des eingesetzten Palladiumkomplexes 0,001 bis 20 Molprozent, bezogen auf die Verbindung der Formel (I) und im Komplex enthaltenes Palladium. Bevorzugt werden 0,05 bis 10 Molprozent eingesetzt.

Bei der zweiten Komponente des Katalysatorsystems, dem Decarboxylierungskatalysator, handelt es sich im erfindungsgemäßen Verfahren um einen Kupferkomplex oder einen Silberkomplex, die präformiert werden oder in situ aus geeigneten Metallsalzen und geeigneten Liganden hergestellt werden.

Die Silberkomplexe können aus Silbersalzen, bevorzugt aus den entsprechenden Fluoriden, Chloriden, Bromiden, Iodiden, 4-Toluolsulfonaten, Methansulfonaten, Trifluormethansulfonaten, Nitraten, Acetaten, Trifluoracetaten, Carbonaten, Oxiden, Sulfaten oder Oxalaten hergestellt werden. Besonders bevorzugt wird zur Herstellung der Silberkomplexe Silbercarbonat eingesetzt.

Alternativ werden Kupferkomplexe als Katalysatoren eingesetzt, die wahlweise aus metallischem Kupfer, Kupfer(I)salzen oder Kupfer(II)salzen und geeigneten Liganden präformiert oder in situ erzeugt werden können. Als Kupfersalze werden dabei bevorzugt die entsprechenden Fluoride, Chloride, Bromide, Iodide, Carboxylate, Trifluormethansulfonate, Oxide, Hydoxide, Carbonate, Sulfate oder Nitrat eingesetzt. Die Kupferkomponenten können auch als Komplexe vorliegen, die einen oder mehrere ungeladene, und wahlweise einzähnige und zweizähnige Liganden tragen z. B. solche aus der Gruppe der Amine, Phosphine, N-heterocyclische Carbene, Nitrile, Olefine und Aromaten. Bevorzugt wird Kupfer(I) oxid (Cu₂O) zur Erzeugung der Kupferkomplexe eingesetzt.

Als Liganden für die Kupfer-oder Silberkomplexe dienen Amine. Bevorzugt werden cyclische Amine als Liganden eingesetzt. Besonders bevorzugt werden chelatisierende cyclische Amine aus der Gruppe Phenanthrolin, Bipyridin und Terpyridin oder ihre substituierten Derivate dafür verwendet. Ganz besonders bevorzugt sind die Verbindungen 1,10-Phenanthrolin, 3,4,7,8-Tetramethyl-1,10-phenanthrolin und 2,2'-Bipyridin.

Im erfindungsgemäßen Verfahren beträgt die Menge der eingesetzten Silbers bzw. Kupfers 0,001 bis 20 Molprozent, bezogen auf die Verbindung der Formel (I). Bevorzugt werden 0,05 bis 10 Molprozent eingesetzt.

Der Ligand des Silber-oder Kupferkomplexes wird in einer Menge von 0,001 bis 30 Molprozent, bezogen auf die eingesetzte Verbindung der Formel (I) eingesetzt. Bevorzugt werden 0,1 bis 10 Molprozent eingesetzt.

Das erfindungsgemäße Verfahren wird bei Temperaturen von 0 °C bis 250 °C, vorzugsweise bei 50 °C bis 200 °C und besonders bevorzugt bei 100°C bis 180 °C durchgeführt.

Das erfindungsgemäße Verfahren wird üblicherweise bei Normaldruck durchgeführt, kann aber auch bei verringertem oder erhöhtem Druck durchgeführt werden.

Das erfindungsgemäße Verfahren wird üblicherweise in Gegenwart eines Lösungsmittels durchgeführt. Verwendet werden können dabei lineare, cyclische oder verzweigte Kohlenwasserstoffe mit C₆-C₂₀ Kohlenstoffatomen wie z.B. Hexane, Heptane, Octane, aromatische Kohlenwasserstoffe wie z.B. Benzol, Toluol, Xylole, Ethylbenzol, Mesitylen, Ether aus der Reihe 1,4-Dioxan, Tetrahydrofuran, Methyltetrahydrofuran, Dibutylether, Methyl-t-butylether, Diisopropylether, Diethylenglycoldimethylether, Diphenylether, Ester wie z.B. Ethylacetat, Butylacetat, Propylencarbonat, Amide der Reihe Dimethylformamid, Diethylformamid, N-Methylpyrrolidon, Dimethylacetamid, schwefelhaltige Verbindungen wie z.B. Dimethylsulfoxid, Sulfolan, Nitrile wie z.B. Acetonitril, Isobutyronitril, Propionitril, oder chlorierte aliphatische oder chlorierte aromatische Kohlenwasserstoffe. Die Lösungsmittel können wahlweise einzeln oder auch als Gemische eingesetzt werden.

Das Lösungsmittel wird möglichst wasserfrei eingesetzt.

Im erfindungsgemäßen Verfahren werden Gemische der Lösungsmittel Mesitylen und N-Methylpyrrolidion bevorzugt, wobei eine Zusammensetzungen der Lösungsmittel Mesitylen und N-Methylpyrrolidion und ein Verhältnis von einem Volumenanteil N-Methylpyrrolidion zu drei Volumenanteilen Mesitylen besonders bevorzugt ist.

Das erfindungsgemäße Verfahren wird so durchgeführt, dass bereits zu Reaktionsbeginn Spuren von Wasser ausgeschlossen werden oder diese während der Reaktion durch übliche Methoden entfernt werden, beispielsweise durch Destillation oder durch Zusatz von Wasser bindenden Mitteln. Bevorzugt wird ein Ausschluss von Feuchtigkeit zu Beginn der Reaktion durch bekannte Methoden.

Das erfindungsgemäße Verfahren wird in gängigen Reaktionsapparaturen unter konventioneller Erwärmung durchgeführt. Eine Reaktionsführung unter Einstrahlung von Mikrowellen in den dafür vorgesehenen Apparaturen ist ebenfalls möglich.

Die gemäß dem erfindungsgemäßen Verfahren hergestellten Verbindungen der Formel (III) werden nach Beendigung der Reaktion vom Reaktionsgemisch, vorzugsweise destillativ und/oder durch Extraktion oder chromatographische Methoden aufgearbeitet.

Die eingesetzten Verbindungen der Formel (IV) sind neu und daher ebenfalls Gegenstand der Erfindung.

Es war überaus überraschend, dass gerade Palladiumkomplexe mit Verbindungen der Formel (IV) als Liganden im Gegensatz zu anderen Phosphinen, auch solchen, die bekanntermaßen für die Aktivierung von Organomesylaten geeignet sind, im erfindungsgemäßen Verfahren eine einzigartige Aktivität zeigen.

Bevorzugte Verbindungen der Formel (IV) sind solche, in denen die Reste R⁷, R⁸, R⁹ und R¹⁰ für Wasserstoff stehen.

Bevorzugte Verbindungen der Formel (IV) sind solche, in denen die Reste R⁵ und R⁶ identisch oder verschieden sind und ausgewählt sind aus der Gruppe Phenyl, Cyclohexyl, Isopropyl, n-Butyl, tert-Butyl Besonders bevorzugt sind die Reste R⁵ und R⁶ Cyclohexyl-Gruppen.

Weitere bevorzugte Verbindungen der Formel (IV) sind solche, bei denen R⁵ und R⁶ in Formel (IV) jeweils für einen Cyclohexylrest stehen , R⁷, R⁸, R⁹, R¹⁰ für Wasserstoff, R¹¹ für einen C₁-C₂₀-Alkylrest stehen und R¹² und R¹³ gemeinsam Bestandteil eines Ringsystems aus der Gruppe C₃-C₆-Alicyclus, C₃-C₂₀-Heterocyclus oder C₆-C₁₀-Aromat sind.

Ein weiterer Gegenstand der Erfindung sind Palladiumkomplexe, die neben Palladium der Oxidationsstufe (0) oder (II) eine der vorstehend beschriebenen Benzimidazolylphosphin-Verbindungen der Formel (IV) als Liganden enthalten.

Der erfindungsgemäße Palladiumkomplex wird aus handelsüblichen Palladiumverbindungen ausgewählt aus der Gruppe der Pd(0)Lₙ-Komplexe, in denen L wahlweise ein oder mehrere ungeladene Liganden aus der Gruppe Triphenylphosphin, Dibenzylidenaceton, Alkenylsilane, und n eine Zahl zwischen 1 und 4 darstellt, oder aus Pd(II)X₂-Salzen, wobei X für Chlorid, Bromid, Iodid, Sulfat, Oxid, Acetat, Trifluoracetat, Acetonat, Hexafluoractetylacetonat, Allyl, Vinyl oder Cinnamyl steht, und die wahlweise durch einen oder mehrere Liganden wie z. B. Alkylnitrile, Phenylnitrile, Phosphine, gesättigte oder ungesättigte N-heterocyclische Carbene, lineare, verzweigte, cyclische Alkene wie z. B. 1,5-Cylooctadien, 1,3,5-Hexatrien, 1,5-Haxadien, Alkenylsiloxane stabilisiert sein können, hergestellt. Oder es werden Pd(0)-Spezies auf üblichen Trägermaterialien wie z.B. Aktivkohle, Aluminiumoxid, Bariumsulfat, Calciumsulfat durch Umsetzung mit einem Benzimidazolylphosphin-Liganden hergestellt.

Bevorzugt werden Palladium(acetylacetonate), Pd(II)acetat, Allylpalladium(II)chlorid und Bis(dibenzylidenaceton)palladium(0) eingesetzt.

Bevorzugt wird der erfindungsgemäße Palladiumkomplex in situ aus einer der genannten Palladiumverbindungen und einer der vorstehend beschriebenen Benzimidazolylphosphin-Verbindungen der Formel (IV) hergestellt.

Erfindungsgemäß werden dabei 1-20 Moläquivalente der Verbindungen der Formel (IV) in Bezug auf die Palladiumkomponente verwendet. Vorzugsweise werden 5-15 Moläquivalente eingesetzt, wobei 2-4 Moläquivalente besonders bevorzugt sind.

Ein weiterer Gegenstand der Erfindung sind die Katalysatoren selbst, die die genannten Kupferkomplexe bzw. Silberkomplexe sowie vorstehend beschriebenen Palladiumkomplexe enthalten, sowie deren Verwendung in einem Verfahren zur decarboxylierenden Kohlenstoff-Kohlenstoff-Verknüpfung von Carbonsäuresalzen mit Aryl-, Heteroaryl-oder Vinylmesylaten.

Die Herstellung der erfindungsgemäßen Benzimidazolylphosphin-Verbindungen der Formel (IV) bzw. das erfindungsgemäße Verfahren zur decarboxylierenden Kohlenstoff-Kohlenstoff- Verknüpfung unter Verwendung dieser Benzimidazolylphosphin-Verbindungen als Liganden der einen Katalysatorkomponente werden durch die folgenden Beispiele veranschaulicht, ohne auf diese eingeschränkt zu sein.

### Beispiele

### Beispiel A: Synthese von 2-(2-(Dicyclohexylphosphino)phenyl)-5,6-dimethyl-1-octyl-1H-benzo[d]imidazol:

### 1.Stufe 2-(2-Bromphenyl)-5,6-dimethyl-1H-benzimidazol:

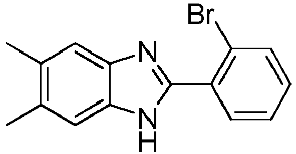

2-Brombenzoesäure (10.1 g, 50 mmol) und 4,5-dimethyl-o-phenylendiamin (7.6, 55 mmol) wurden in Polyphosphorsäure 6h bei 150 °C erhitzt. Die Reaktionsmischung wurde auf Eis gegossen und über Nacht im Kühlschrank stehen gelassen. Der ausgefallene Niederschlag wurde filtriert, in eine wässrige Na₂CO₃-Lösung (0.5 M, 650 mL) gegebenen und 30 min gerührt. Der erhaltene gelbe Feststoff wurde filtriert, in heißem wässrigem Methanol (50% Vol., 200 mL) gelöst und innerhalb von 1h in eine wässrige Na₂CO₃-Lösung (0.5 M, 650 mL) getropft. Der erhaltene Feststoff wurde filtriert, getrocknet, und das Produkt wurde als gelber Feststoff (14.6 g, 48.5 mmol, 97%) erhalten.

### 2.Stufe: 2-(2-Bromphenyl)-5,6-dimethyl-1-octyl-1H-benzimidazol:

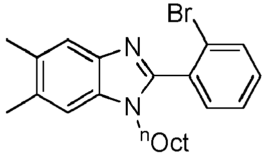

2-(2-Bromphenyl)-5,6-dimethyl-1H-benzimidazol (2.1 g, 7.0 mmol) wurde in wasserfreiem THF (30 mL) gelöst, auf 0 °C gekühlt und Natriumhydrid (60%, Suspension in Mineralöl, 336 mg, 14.0 mmol) zugeben. Nach beendeter Gasentwicklung wurde 1-Bromoctan (2.7 g, 14.0 mmol) so zugetropft, dass die Reaktionstemperatur 5 °C nicht übersteig. Nach vollständigem Umsatz wurde die Reaktionsmischung mit gesättigter wässriger NH₄Cl-Lösung (30 mL) versetzt und mit Ethylacetat extrahiert (4 x 30 mL). Die vereinigten organischen Phasen wurden mit Wasser (100 mL) gewaschen, mit Na₂SO₄, getrocknet, filtriert und konzentriert. Der verbliebene Rückstand wurde säulenchromatographisch (SiO₂, 30% Ethylacetat in Hexan) gereinigt und das Produkt wurde als gelbes Öl (2.7 g, 6.5 mmol, 93%) erhalten.

### 3.Stufe: 2-(2-(Dicyclohexylphosphino)phenyl)-5,6-dimethyl-1-octyl-1H-benzo[d]imidazol:

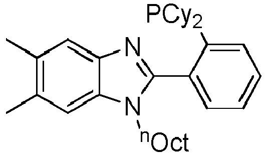

2-(2-Bromphenyl)-5,6-dimethyl-1-octyl-1H-benzimidazol (2.5 g, 6.1 mmol) wurde unter Stickstoffatmosphäre in wasserfreiem THF (30 mL) gelöst, in einem Kältebad auf -78 °C gekühlt, *n*-Butyllithium-Lösung (4.2 mL, 1.6 M, 6.7 mmol) zugetropft und 30 min gerührt. Eine Lösung aus Chlordicyclohexylphosphin (1.48 mL, 6.7 mmol) in THF (10 mL) wurde zugegeben und 1h bei -78 °C gerührt. Die Reaktionsmischung wurde auf Raumtemperatur erwärmt, über Nacht gerührt und durch Celite filtriert. Die Filterhilfe wurde mit Ethylacetat gewaschen und die vereinigten Filtrate wurden konzentriert. Der verbliebene Rückstand wurde säulenchromatographisch (SiO₂, 15% Ethylacetat in Hexan) gereinigt und das Produkt wurde als gelbes Öl (2.8 g, 5.3 mmol, 87%) erhalten.

### Besipiel B: Synthese von 5,6-Dichlor-2-(2-(dicyclohexylphosphino)phenyl)-1-methyl-1H-benzo[d]imidazol:

### 1.Stufe: 2-(2-Bromophenyl)-5,6-dichloro-1H-benzimidazole:

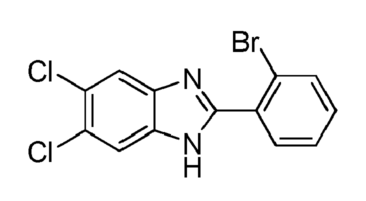

2-Brombenzoesäure (3.11 g, 15.0 mmol) and 4,5-Dichlor-o-phenylendiamin (2.92, 16.5 mmol) wurden 6h bei 150 °C in Polyphosphorsäure gerührt. Die Reaktionsmischung wurde auf Eis gegossen und über Nacht im Kühlschrank stehen gelassen. Der ausgefallene Niederschlaf wurde filtriert, in eine wässrige Na₂CO₃-Lösung (0.5 M, 650 mL) gegebenen, 30 min gerührt. Der erhaltene gelbe Feststoff wurde filtriert, in heißem wässrigem Methanol (50% Vol., 200 mL) gelöst und innerhalb von 1 h in eine wässrige Na₂CO₃-Lösung (0.5 M, 650 mL) getropft. Der erhaltene Feststoff wurde filtriert, getrocknet, und das Produkt wurde als farbloser Feststoff (4.5 g, 13.2 mmol, 88%) erhalten.

### 2.Stufe: 2-(2-Bromphenyl)-5,6-dichlor-1-methyl-1H-benzimidazol:

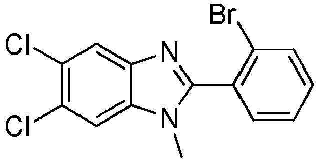

2-(2-Bromphenyl)-5,6-dichlor-1H-benzimidazol (2.4 g, 7.0 mmol) wurde unter Stickstoffatmosphäre in wasserfreiem THF gelöst, auf 0 °C gekühlt, und KHMDS-Lösung (0.5 M in Toluol, 14.7 mL, 7.4 mmol) zugegeben. Die erhltene Lösung wurde 1.5 h bei Raumtemperatur gerührt, auf 0°C gekühlt und Methyliodid (0.46 mL, 7.4 mmol) zugetropft. Die erhaltene Lösung wurde 2 h bei Raumtemperatur gerührt, gesättigte wässrige NH₄Cl-Lösung (50 mL) wurde zugegeben und mit Ethylacetat (3 x 30 mL) extrahiert. Die vereinigten organischen Phasen wurden mit gesättigert wässriger Kochsalzlösung (50 mL) gewaschen, mit MgSO₄ getrocknet, filtriert und konzentriert. Das Produkt wurde als gelblicher Feststoff (2.4 g, 6.74 mmol, 96%) erhalten.

### 3.Stufe: 5,6-Dichlor-2-(2-(dicyclohexylphosphino)phenyl)-1-methyl-1H-benzo[d]imidazol:

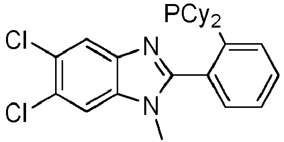

2-(2-Bromphenyl)-5,6-dichlor-1-methyl-1H-benzimidazol (2.63 g, 7.3 mmol) wurde unter Stickstoffatmosphäre in wasserfreiem THF (30 mL) gelöst, in einem Kältebad auf -78 °C gekühlt, *n*-Butyllithium-Lösung (4.8 mL, 1.6 M, 7.7 mmol) zugetropft und 30 min gerührt. Eine Lösung aus Chlordicyclohexylphosphin (1.74 mL, 7.7 mmol) in THF (10 mL) wurde zugetropft und 1 h bei -78 °C gerührt. Die Reaktionsmischung wurde auf Raumtemperatur erwärmt, über Nacht gerührt und durch Celite filtriert. Die Filterhilfe wurde mit Ethylacetat gewaschen und die vereinigten Filtrate wurden konzentriert. Der verbliebene Rückstand wurde aus Acetonitril umkristallisiert und das Produkt als ein farbloser Feststoff erhalten (2.67 g, 5.64 mmol, 77%).

### In analoger Weise wurden auch die folgenden Verbindungen hergestellt:

### Beispiel C: 2-(2-(Dicyclohexylphosphino)phenyl)-1,5,6-trimethyl-1H-benzo[d]imidazol:

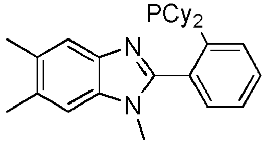

### Beispiel D: 2-(2-(Dicyclohexylphosphino)phenyl)-1-isopropyl-5,6-dimethyl-1H-benzo[d]imidazol:

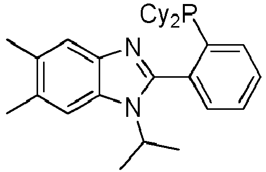

### Beispiel E: 1-Butyl-2-(2-(dicyclohexylphosphino)phenyl)-5,6-dimethyl-1H-enzo[d]imidazol:

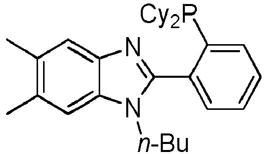

### Beispiel F: 2-(2-(Dicyclohexylphosphino)phenyl)-5,6-dimethyl-1-octadecyl-1H-benzo[d]imidazol:

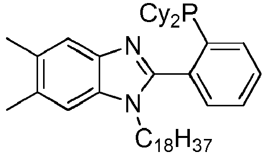

### Beispiel G: 2-(2-(Dicyclohexylphosphino)phenyl)-1-isopropyl-1H-benzo[d]imidazol:

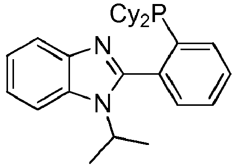

### Beispiel H: 2-(2-(Dicyclohexylphosphino)phenyl)-1-(methoxymethyl)-1H-benzoimidazol:

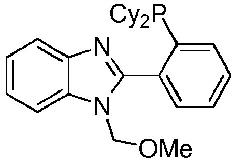

### Beispiel I: 2-(2-(Di-tert-butylphosphino)phenyl)-1-methyl-1H-benzoimidazol:

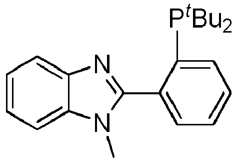

### Beispiel J: 2-(2-(Diphenylphosphino)phenyl)-1-methyl-1H-benzoimidazol:

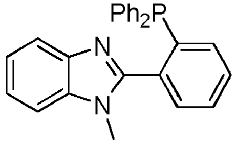

### Beispiel K: 2-(2-(Dicyclohexylphosphino)phenyl)-1-phenyl-1H-benzoimidazol:

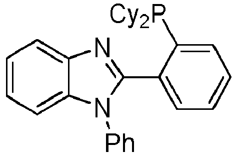

### Das N-Phenylderivat wurde ausgehend von N-Phenyl-o-phenylenediamin synthetisiert.

### Beispiel L: 2-(2-(Dicyclohexylphosphino)-3,4,5-trimethoxyphenyl)-1-methyl-1H-benzo[d]imidazol:

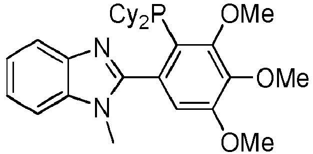

### Beispiel M: 2-(2-(Dicyclohexylphosphino)phenyl)-5,6-dimethoxy-1-methyl-1H-benzo[d]imidazol:

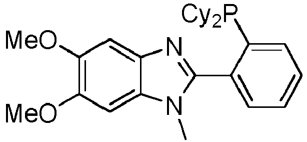

### Beispiel N: 2-(2-(Dicyclohexylphosphino)phenyl)-1-methyl-4,5,6,7-tetrahydro-1H-benzo[d]imidazol:

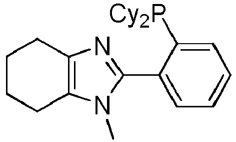

### Beispiel O: 2-(2-(Dicyclohexylphosphino)phenyl)-1-octyl-4,5,6,7-tetrahydro-1H-benzo[d]imidazol:

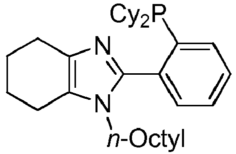

### Beispiel 1: 2-(2-Nitrophenyl)naphthalin

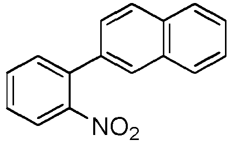

In einem ausgeheiztem Schlenk-Gefäß wurden 103 mg [0.5 mmol] Kalium-2-nitrobenzoat, 167 mg [0.75 mmol] 2-Naphthylmethanesulfonat, 14.4 mg [0.025 mmol] Pd(dba)₂ (= Bis(dibenylidenaceton)palladium), 31.8 mg [0.06 mol] **1-n-Octyl-2-(2-(dicyclohexylphosphino)phenyl)-5,6-dimethyl-1H-benzimidazol** (hergestellt analog Beispiel F), 1.81 mg [0.0125 mmol] Kupfer(I)oxid, 5.91 mg [0.025 mmol] 3,4,7,8-Tetramethyl-1,10-phenanthrolin, 1 mL NMP, 3 mL Mesitylen vorgelegt. Das Reaktionsgefäß wurde dreimal evakuiert und mit Stickstoff befüllt, und bis zum vollständigen Reaktionsumsatz (1 bis 12 Stunden) bei 170 °C gerührt. Nach Abkühlen auf Raumtemperatur wurde das Reaktionsgemisch mit Ethylacetat verdünnt. Die resultierende Lösung wurde nacheinander mit je 20 ml Wasser, gesättigter wässr. NaHCO₃-Lsg. und gesättigter wässr. NaCl-Lösung gewaschen, über MgSO₄ getrocknet, filtriert und im Vakuum eingeengt. Das Rohprodukt wurde chromatographisch gereinigt (Kieselgel, Hexan/ Essigester) und 2-(2-Nitrophenyl)naphthalin wurde in einer Ausbeute von 86% als gelber Feststoff erhalten.

### Beispiel 2: 2-(5-Methyl-2-nitrophenyl)naphthalin

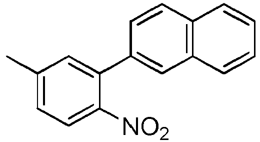

Analog zu Beispiel 1 wurden aus 110 mg [0.5 mmol] Kalium-5-methyl-2-nitrobenzoat und 167 mg [0.75 mmol] 2-Naphthylmesylat 232 mg das Produkt in einer Ausbeute von 88% als gelber Feststoff erhalten.

### Beispiel 3: 2-(5-Methoxy-2-nitrophenyl)naphthalin

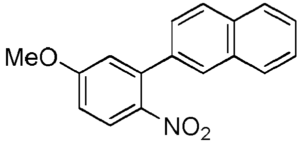

Analog zu Beispiel 1 wurden aus 118 mg [0.5 mmol] Kalium-5-methoxy-2-nitrobenzoat und 167 mg [0.75 mmol] 2-Naphthylmesylat 236 mg des Produkts in einer Ausbeute bin 85% als gelber Feststoff erhalten.

### Beispiel 4: 2-(3-Methyl-2-nitrophenyl)naphthalin

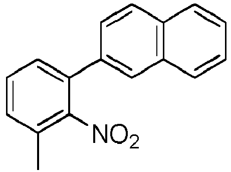

Analog zu Beispiel 1 wurden aus 110 mg [0.5 mmol] Kalium-3-methyl-2-nitrobenzoat und 167 mg [0.75 mmol] 2-Naphthylmesylat 222 mg des Produktes in einer Ausbeute von 84% als gelber Feststoff erhalten.

### Beispiel 5: 2-(5-Fluor-2-nitrophenyl)naphthalin

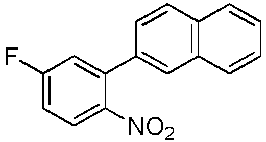

Analog zu Beispiel 1 wurden aus 230 mg [1.0 mmol] Kalium-5-fluor-2-nitrobenzoat und 115 mg [0.5 mmol] 2-Naphthylmesylat 214 mg des Produktes in einer Ausbeute von 85% als gelber Feststoff erhalten.

### Beispiel 6: 2-(2-Methyl-6-nitrophenyl)naphthalin

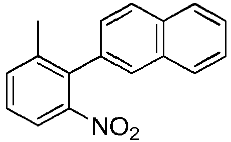

Analog zu Beispiel 1 wurden aus 220 mg [1.0 mmol] Kalium-6-methyl-2-nitrobenzoat und 115 mg [0.5 mmol] 2-Naphthylmesylat 132 mg des Produktes in einer Ausbeute von 50% als gelber Feststoff erhalten.

### Beispiel 7: 3-Methyl-2-(naphthalin-2-yl)thiophen

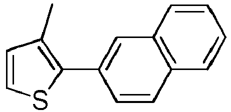

Analog zu Beispiel 1 wurden aus 91 mg [0.5 mmol] Kalium-3-methylthiophen-2-carboxylat und 167 mg [0.75 mmol] 2-Naphthylmesylat 100 mg des Produktes in einer Ausbeute von 45% als farbloser Feststoff erhalten.

### Beispiel 8: 3-Methyl-2-(naphthalin-2-yl)benzo[b]thiophen

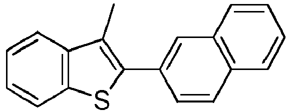

Analog zu Beispiel 1 wurden aus 115 mg [0.5 mmol] Kalium-3-methylbenzo[b]thiophen-2-carboxylat und 167 mg [0.75 mmol] 2-Naphthylmesylat 200 mg des Produktes in einer Ausbeute von 73% als farbloser Feststoff erhalten.

### Beispiel 9: 3-Methyl-2-(naphthalin-2-yl)benzofuran

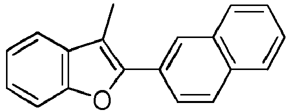

Analog zu Beispiel 1 wurden aus 107 mg [0.5 mmol] Kalium-3-methylbenzofuran-2-carboxylat und 167 mg [0.75 mmol] 2-Naphthylmesylat 130 mg des Produktes in einer Ausbeute von 50% als farbloser Feststoff erhalten.

### Beispiel 10: 1-Methyl-2-(naphthalin-2-yl)-1H-indol

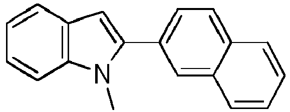

Analog zu Beispiel 1 wurden aus 107 mg [0.5 mmol] Kalium-1-methyl-1H-indol-2-carboxylat und 167 mg [0.75 mmol] 2-Naphthylmesylat 160 mg des Produktes in einer Ausbeute von 62% als farbloser Feststoff erhalten.

### Beispiel 11: 1-(2-Nitrophenyl)naphthalin

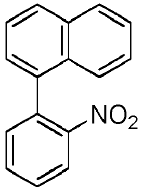

Analog zu Beispiel 1 wurden aus 144 mg [0.7 mmol] Kalium-2-nitrobenzoat und 233 mg [1.05 mmol] 1-Naphthylmesylat 45 mg des Produktes in einer Ausbeute von 27% als gelber Feststoff erhalten.

### Beispiel 12: 9-(2-Nitrophenyl)phenanthren

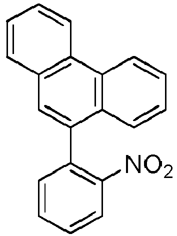

Analog zu Beispiel 1 wurden aus 103 mg [0.5 mmol] Kalium-2-nitrobenzoat und 211 mg [0.75 mmol] Phenanthren-9-ylmesylat 39 mg des Produktes in einer Ausbeute von 26% als gelber Feststoff erhalten.

### Beispiel 13: 4'-Methyl-2-nitro-1,1'-biphenyl

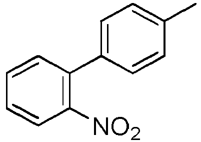

Analog zu Beispiel 1 wurden aus 103 mg [0.5 mmol] Kalium-2-nitrobenzoat und 186 mg [1.0 mmol] 4-Tolylmesylat 30 mg des Produktes in einer Ausbeute von 28% als gelbes Öl erhalten.

### Beispiel 14: 3'-Methoxy-2-nitro-1,1'-biphenyl

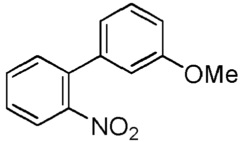

Analog zu Beispiel 1 wurden aus 103 mg [0.5 mmol] Kalium-2-nitrobenzoat und 202 mg [1.0 mmol] 3-Methoxyphenylmesylat 40 mg des Produktes in einer Ausbeute von 35% als gelbes Öl erhalten.

### Beispiel 15: 3-(2-Nitrophenyl)-1,2-dihydronaphthalin

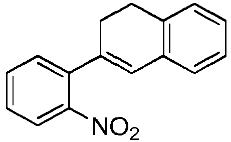

In einem ausgeheiztem Schlenk-Gefäß wurden 103 mg [0.5 mmol] Kalium2-nitrobenzoat, 167 mg [0.75 mmol] 3,4-Dihydronaphthalin-2-ylmethanesulfonat, 5.73 mg [0.025 mmol] Pd(OAc)₂, 28.4 mg [0.06 mol] **1-Methyl-2-(2-(dicyclohexylphosphino)phenyl)-5,6-dimethyl-1H-benzimidazol** (hergestellt gemäß Beispiel M), 1.81 mg [0.0125 mmol] Kupfer(I)oxid, 3.94 mg [0.025 mmol] 2,2'-Bipyridin, 1 mL NMP, 3 mL Mesitylen vorgelegt. Das Reaktionsgefäß wurde dreimal evakuiert und mit Stickstoff befüllt, und bis zum vollständigen Umsatz (1 bis 5 Stunden) bei 170 °C gerührt. Nach Abkühlen auf Raumtemperatur wurde das Reaktionsgemisch mit Ethylacetat verdünnt. Die resultierende Lösung wurde nacheinander mit je 20 ml Wasser, gesättigter wässr. NaHCO₃-Lsg. und gesättigter wässr. NaCl-Lösung gewaschen, über MgSO₄ getrocknet, filtriert und im Vakuum eingeengt. Das Rohprodukt wurde chromatographisch gereinigt (Kieselgel, Hexan/ Essigester) und 3-(2-Nitrophenyl)-1,2-dihydronaphthalin in einer Ausbeute von 86% als gelbes Öl erhalten.

### Beispiel 16: 3-(5-Methyl-2-nitrophenyl)-1,2-dihydronaphthalin

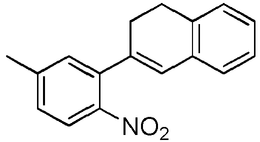

Analog zu Beispiel 15 wurden aus 132 mg [0.6 mmol] Kalium-5-methyl-2-nitrobenzoat und 208 mg [0.9 mmol] 3,4-Dihydronaphthalin-2-ylmethanesulfonat 130 mg des Produktes in einer Ausbeute von 82% als gelbes Öl erhalten.

### Beispiel 17: 3-(5-Methoxy-2-nitrophenyl)-1,2-dihydronaphthalin

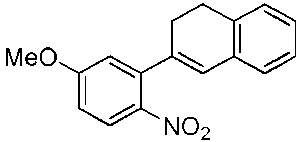

Analog zu Beispiel 15 wurden aus 141 mg [0.6 mmol] Kalium-5-methoxy-2-nitrobenzoat und 208 mg [0.9 mmol] 3,4-Dihydronaphthalin-2-ylmethanesulfonat 142 mg des Produktes in einer Ausbeute von 84% als gelbes Öl erhalten.

### Beispiel 18: 3-(4,5-Dimethoxy-2-nitrophenyl)-1,2-dihydronaphthalin

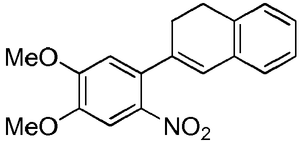

Analog zu Beispiel 15 wurden aus 159 mg [0.6 mmol] Kalium-4,5-dimethoxy-2-nitrobenzoat und 208 mg [0.9 mmol] 3,4-Dihydronaphthalin-2-ylmethanesulfonat 155 mg des Produktes in einer Ausbeute von 83% als gelbes Öl erhalten.

### Beispiel 19: 3-(3-Methyl-2-nitrophenyl)-1,2-dihydronaphthalin

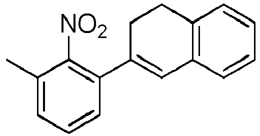

Analog zu Beispiel 15 wurden aus 136 mg [0.6 mmol] Kalium-3-methyl-2-nitrobenzoat und 208 mg [0.9 mmol] 3,4-Dihydronaphthalin-2-ylmethanesulfonat 136 mg des Produktes in einer Ausbeute von 85% als gelbes Öl erhalten.

### Beispiel 20: 3-(5-Fluor-2-nitrophenyl)-1,2-dihydronaphthalin

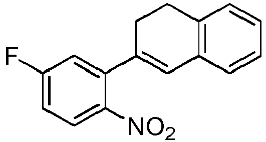

Analog zu Beispiel 15 wurden aus 159 mg [0.6 mmol] Kalium-5-fluor-2-nitrobenzoat und 208 mg [0.9 mmol] 3,4-Dihydronaphthalin-2-ylmethanesulfonat 129 mg des Produktes in einer Ausbeute von 80% als gelbes Öl erhalten.

### Beispiel 21: 3-(Pentafluorphenyl)-1,2-dihydronaphthalin

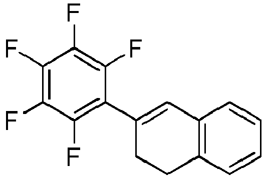

Analog zu Beispiel 15 wurden aus 175 mg [0.7 mmol] Kalium-2,3,4,5,6-pentafluorbenzoat und 243 mg [1.05 mmol] 3,4-Dihydronaphthalin-2-ylmethanesulfonat 46 mg des Produktes in einer Ausbeute von 22% als gelbes Öl erhalten.

### Beispiel 22: 3-(2-Methyl-6-nitrophenyl)-1,2-dihydronaphthalin

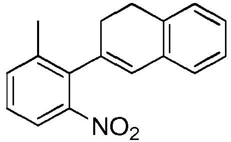

Analog zu Beispiel 15 wurden aus 110 mg [0.5 mmol] Kalium-6-methyl-2-nitrobenzoat und 168 mg [0.75 mmol] 3,4-Dihydronaphthalen-2-ylmethanesulfonat 56 mg des Produktes in einer Ausbeute von 42% als gelbes Öl erhalten.

### Beispiel 23: 2-(3,4-Dihydronaphthalin-2-yl)-3-methylbenzo[b]thiophen

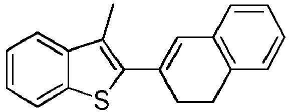

Analog zu Beispiel 15 wurden aus 161 mg [0.7 mmol] Kalium-3-methylbenzo[b]thiophen-2-carboxylat und 243 mg [1.05 mmol] 3,4-Dihydronaphthalin-2-ylmethanesulfonat 105 mg des Produktes in einer Ausbeute von 54% als gelbes Öl erhalten.

### Beispiel 24: (2-(2-Nitrophenyl)ethen-1,1-diyl)dibenzol

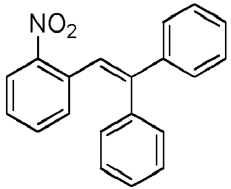

Analog zu Beispiel 15 wurden aus 103 mg [0.5 mmol] Kalium-2-nitrobenzoat und 206 mg [0.75 mmol] 2,2-Diphenylvinylmethanesulfonat 125 mg des Produkts in einer Ausbeute von 83% als gelbes Öl erhalten.

### Beispiel 25: (E)-o-Nitrostilben

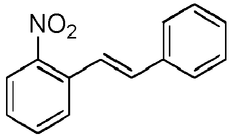

Analog zu Beispiel 15 wurden aus 103 mg [0.5 mmol] Kalium-2-nitrobenzoat und 149 mg [0.75 mmol] Styrylmethanesulfonat 97 mg des Produktes in einer Ausbeute von 86% als gelbes Öl erhalten.

### Beispiel 26: 1-Nitro-2-(1-phenylbut-1-en-1-yl)benzol

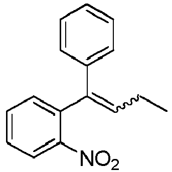

Analog zu Beispiel 15 wurden aus 206 mg [1.0 mmol] Kalium-2-nitrobenzoat und 113 mg [0.5 mmol] 1-Phenylbut-1-en-1-ylmethanesulfonat 50 mg des Produktes in einer Ausbeute von 40% als gelbes Öl erhalten.

### Beispiel 27: (2-(2-Nitrophenyl)prop-1-en-1,1-diyl)dibenzol

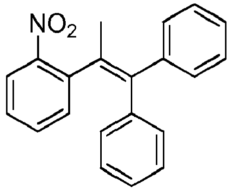

Analog zu Beispiel 15 wurden aus 103 mg [0.5 mmol] Kalium-2-nitrobenzoat und 223 mg [0.75 mmol] 1,1-Piphenylprop-1-en-2-ylmethanesulfonat 100 mg des Produktes in einer Ausbeute von 63% als gelbes Öl erhalten.

### Beispiel 28: (E)-1-(1-(4-Methoxyphenyl)prop-1-en-2-yl)-2-nitrobenzol

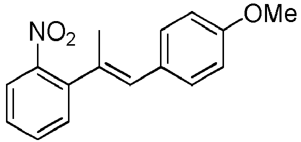

Analog zu Beispiel 15 wurden aus 103 mg [0.5 mmol] Kalium-2-nitrobenzoate und 187 mg [0.75 mmol] (E)-1-(4-Methoxyphenyl)prop-1-en-2-ylmethanesulfonat 99 mg des Produktes in einer Ausbeute von 73% als gelbes Öl erhalten.

### Beispiel 29: o-Nitrostyrol

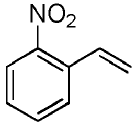

Analog zu Beispiel 15 wurden aus 206 mg [1.0 mmol] Kalium-2-nitrobenzoat und 366 mg [3.0 mmol] Vinylmethansulfonat 81 mg des Produktes in einer Ausbeute von 54% als gelbes Öl erhalten.

### Beispiel 30: Methyl 2-(2-nitrophenyl)cyclopent-1-encarboxylat

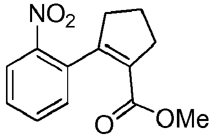

Analog zu Beispiel 15 wurden aus 440 mg [2.1 mmol] Kalium-2-nitrobenzoat und 318 mg [1.4 mmol] Methyl 2-((methylsulfonyl)oxy)cyclopent-1-encarboxylat 90 mg des Produktes in einer Ausbeute von 26% als gelbes Öl erhalten.

## Patentansprüche

1. Verfahren zur Herstellung von Verbindungen der Formel (III)
R¹-R^{X} (III)
in der R¹
für Wasserstoff oder einen über ein sp2-hybridisiertes Kohlenstoffatom gebundenen unsubstituierten oder substituierten Rest aus der Gruppe C₆-C₂₀-Aryl, C₃-C₂₀-Heteroaryl oder C₂- C₂₀- Alkenyl steht,
und
R^{X} für 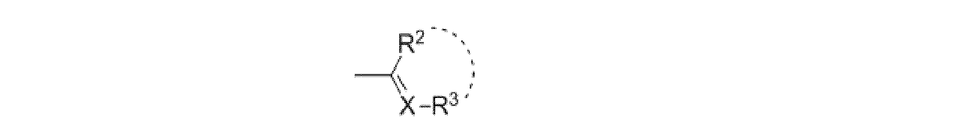 steht, worin X wahlweise für ein Stickstoffatom oder für einen Rest C-R⁴ steht und
R³ für einen organischen Rest aus der Gruppe C₁-C₁₀-Alkyl, C₂-C₂₀-Alkenyl, unsubstituiertes oder substituiertes C₆-C₂₀-Aryl oder für unsubstituiertes oder substituiertes Heteroaryl aus der Gruppe Pyridin, Pyrimidin, Pyridazin, Pyrazin, Triazin, Tetrazin, Pyrrol, Pyrazol, Isoxazol, Imidazol, Oxazol, Thiazol, Thiophen, Furan, stehen und
R² und R⁴ unabhängig voneinander für Wasserstoff stehen oder die gleiche Bedeutung, wie für R³ angegeben, besitzen
oder R² und X-R³ Bestandteil eines unsubstituierten oder substituierten (hetero)cyclischen Ringsystems aus der Gruppe Benzol, Naphthalin, Dihydronaphthalin, Cyclopent-1-en, Phenanthren, Pyridin, Pyrimidin, Pyridazin, Pyrazin, Triazin, Tetrazin, Pyrrol, Pyrazol, Isoxazol, Imidazol, Oxazol, Thiazol, Thiophen, und Furan sind,
durch Umsetzung von Verbindungen der Formel (I)
R¹-COOM (I)
in der M für ein positiv geladenes Ion aus der Gruppe der Alkalimetalle Li, Na, K, Rb, Cs, oder ein Ammoniumkation steht, und
R¹ die für Formel (III) angegebene Bedeutung besitzt,
mit Verbindungen der Formel (II)
R^{X}-O-SO₂-ALK (II)
in der ALK für C₁-C₂₀-Alkyl, vorzugsweise Methyl steht
und
R^{X} die für Formel (III) angegebenen Bedeutung besitzt,
in Gegenwart eines Katalysators, der enthält
- zumindest einen Kupferkomplex, enthaltend ein Amin, oder alternativ zumindest einen Silberkomplex , enthaltend ein Amin, und
- zumindest einen Palladiumkomplex, vorzugsweise in den Oxidationsstufen (II) oder (0), enthaltend einen Benzimidazolylphosphin-Liganden, gemäß Formel (IV). 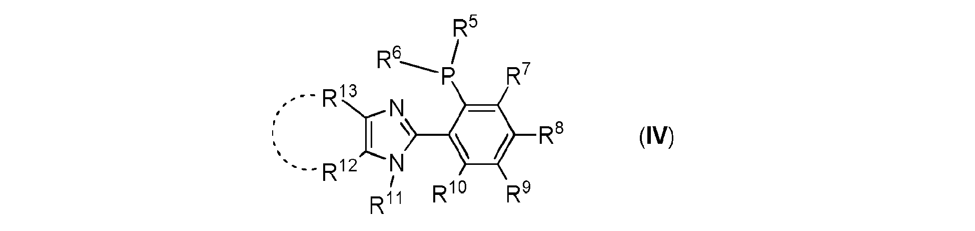
in welcher
R⁵ und R⁶ unabhängig voneinander stehen für einen Rest aus der Gruppe C₁-C₂₀-Alkyl, C₂-C₂₀-Vinyl-, C₆-C₂₀-Aryl-, oder C₃-C₂₀-Heteroaryl, C₁-C₂₀-Alkoxy, C₆-C₂₀-Aryloxy, (C₁-C₂₀-Alkyl)-amino, (C₆-C₂₀-Aryl)amino, Di-(C₆-C₂₀-Aryl)amino oder
R⁵ und R⁶ gemeinsam für C₂-C₁₈-Alkandiyl stehen und
R⁷, R⁸, R⁹, R¹⁰ unabhängig voneinander für einen Rest aus der Gruppe Wasserstoff, Formyl, Hydroxy, Amino, Cyano, Nitro, Fluor, Chlor, Brom, Iod, C₁-C₂₀-Alkyl, C₂-C₂₀-Vinyl-, C₆-C₂₀-Aryl-, oder C₃-C₂₀-Heteroaryl stehen und
R¹¹ für einen Rest aus der Gruppe Wasserstoff, C₁-C₂₀-Alkyl, C₂-C₂₀-Vinyl-, C₆-C₂₀-Aryl-, oder C₃-C₂₀-Heteroaryl und
R¹² und R¹³ unabhängig voneinander für einen Rest aus der Gruppe Wasserstoff, Carboxy, Cyano, Nitro, Fluor, Chlor, Brom, Iod, C₁-C₂₀-Alkyl, C₂-C₂₀-Vinyl-, C₆-C₂₀-Aryl-, oder C₃-C₂₀-Heteroaryl stehen oder gemeinsam Bestandteil eines nichtaromatischen, aromatischen oder heteroaromatischen Ringsystems sind.

2. Verfahren nach Anspruch 1, **dadurch gekennzeichnet, dass** die Verbindungen der Formel (I) Kaliumsalze aus der Gruppe 2-Nitrobenzoesäure, 4-Methyl-2-Nitrobenzoesäure, 4-Methoxy-2-Nitrobenzoesäure, 3-Methyl-2- Nitrobenzoesäure, 4-Fluor-2- Nitrobenzoesäure, 6-Methyl-2-Nitrobenzoesäure, 2-Anissäure, 3-Methyl-2-thiophencarbonsäure, 3-Methyl-2-benzothiphencarbonsäure, 3-Methy-2-benzofurancarbonsäure, N-Methyl-2-inolcarbonsäure, 3-Nitrobenzoesäure, 4,5-Dimethoxybenzoesäure, 2,2,4,5,6- Pentafluorbenzoesäoure und Zimtsäure sind.

3. Verfahren nach einem der Ansprüche 1 bis 2, **dadurch gekennzeichnet, dass** die Verbindungen der Formel (II) Mesylate aus der Gruppe 2-Naphtyl-, 1-Naphtyl-, 9-Phenanthryl-, 4-Toluyl-, 3-Anisyl-, 3,4- Dehydronaphtyl-, 1,1-Diphenylethenyl-, Styryl, Vinyl und Alkenylmesylat sind.

4. Verfahren nach einem der Ansprüche 1 bis 3, **dadurch gekennzeichnet, dass** das Molverhältnis zwischen den Verbindungen der Formel (I) und den Verbindungen der Formel (II) im Bereich von 1:1 bis 1:10 liegt.

5. Verfahren nach einem der Ansprüche 1 bis 4, **dadurch gekennzeichnet, dass** die Menge des Palladiumkomplexes 0,001 bis 20 Molprozent, bezogen auf die Verbindung der Formel (I), beträgt.

6. Verfahren nach einem der Ansprüche 1 bis 5, **dadurch gekennzeichnet, dass** bei Einsatz eines Silberkomplexes dieser aus Silbercarbonat und bei Einsatzes eines Kupferkomplexes dieser aus Kupfer(I)oxid jeweils in situ im Reaktionsgemisch unter Zugabe des jeweiligen Amins als Ligand hergestellt wird.

7. Verbindungen der Formel (IV) 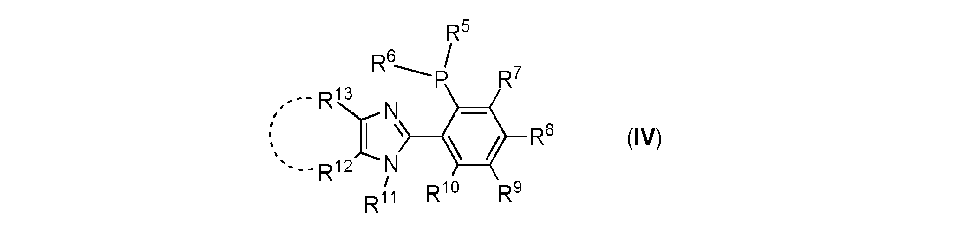 in welchen
R⁵ und R⁶ unabhängig voneinander für einen Rest aus der Gruppe, C₁-C₂₀-Alkyl, C₂-C₂₀- Vinyl-, C₆-C₂₀-Aryl-, oder C₃-C₂₀-Heteroaryl, C₁-C₂₀-Alkoxy, C₆-C₂₀-Aryloxy, (C₁-C₂₀-Alkyl)- amino, (C₆-C₂₀-Aryl)amino, Di-(C₆-C₂₀-Aryl)amino stehen oder
R⁵ und R⁶ gemeinsam für C₂-C₁₈-Alkandiyl stehen und
R⁷, R⁸, R⁹, R¹⁰ unabhängig voneinander für einen Rest aus der Gruppe Wasserstoff, Formyl, Hydroxy, Amino, Cyano, Nitro, Fluor, Chlor, Brom, Iod, C₁-C₂₀-Alkyl, C₂-C₂₀-Vinyl-, C₆-C₂₀-Aryl- und C₃-C₂₀-Heteroaryl stehen und
R¹¹ für einen Rest aus der Gruppe Wasserstoff, C₁-C₂₀₋Alkyl, C₁-C₂₀-Alkoxy, C₂-C₂₀-Vinyl-, C₆-C₂₀-Aryl-, oder C₃-C₂₀-Heteroaryl steht und
R¹² und R¹³ unabhängig voneinander für einen Rest aus der Gruppe Wasserstoff, Carboxyl, Cyano, Nitro, Fluor, Chlor, Brom, Iod, C₁-C₂₀-Alkyl, C₁-C₂₀-Alkoxy, C₂-C₂₀-Vinyl-, C₆-C₂₀-Aryl- und C₃-C₂₀-Heteroaryl stehen oder gemeinsam Bestandteil eines organischen nichtaromatischen, aromatischen oder heteroaromatischen Ringsystems sind.

8. Verbindungen der Formel (IV) nach Anspruch 7 , **dadurch gekennzeichnet, dass** R⁵ und R⁶ in Formel (IV) jeweils für einen Cyclohexylrest stehen.

9. Verbindungen der Formel (IV) nach einem der Ansprüche 7 bis 8 , **dadurch gekennzeichnet, dass** R⁵ und R⁶ in Formel (IV) jeweils für einen Cyclohexylrest, R⁷, R⁸, R⁹, R¹⁰ für Wasserstoff und R¹¹ für einen C₁-C₁₀-Alkylrest steht und R¹² und R¹³ gemeinsam Bestandteil eines Ringsystems aus der Gruppe C₃-C₆-Alicyclus, C₃-C₂₀-Heterocyclus oder C₆-C₁₀-Aromat sind.

10. Palladiumkomplex, enthaltend neben Palladium der Oxidationsstufe (0) oder (II) eine Verbindung der Formel (IV) gemäß einem der Ansprüche 7 bis 9.

11. Katalysator, der enthält
- zumindest einen Kupferkomplex, enthaltend ein Amin, oder alternativ zumindest einen Silberkomplex, enthaltend ein Amin, und
- zumindest einen Palladiumkomplex, vorzugsweise in den Oxidationsstufen (II) oder (0), enthaltend einen Benzimidazolylphosphin-Liganden, gemäß Formel (IV). 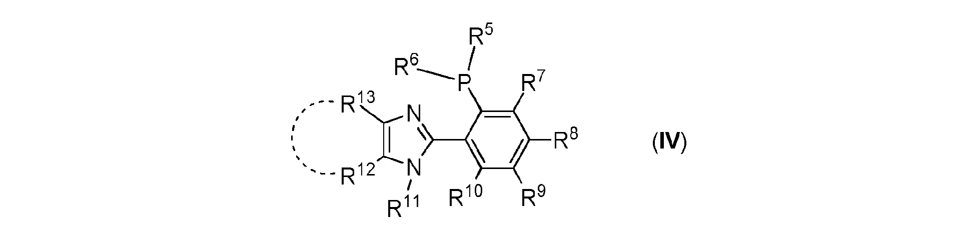 in welcher R⁵ bis R¹³ die oben angegebene Bedeutung besitzen.

12. Verwendung einer Verbindung gemäß einem der Ansprüche 7 bis 9 oder eines Katalysators gemäß Anspruch 11 in einem Verfahren zur decarboxylierenden Kohlenstoff-Kohlenstoff-Verknüpfung von Carbonsäuresalzen mit Aryl-, Heteroaryl-oder Vinylmesylaten.
